# EUROPÄISCHE PATENTANMELDUNG

(11) **EP 1 875 896 A1**
(43) Veröffentlichungstag der Anmeldung: **09.01.2008**
(21) Anmeldenummer: 06116454.7
(22) Anmeldetag: 30.06.2006
(51) Int. Cl.: A61K 9/20, A61K 31/205, A61K 31/685

(54) **Solubilisatformulierungen**

(71) Anmelder: Langhoff, Gertrud, 50169 Kerpen (DE)
(72) Erfinder: Langhoff, Gertrud, Elsdorf 50189 (DE)
(74) Vertreter: Weber, Thomas

(57) **Zusammenfassung**

Die Erfindung betrifft Zusammensetzungen enthaltend ein oder mehrere Phospholipid(e), einen Micellstabilisator, bestehend aus Carnitin und/oder einem physiologisch verträglichen Carnitin-Derivat und/oder Polyolen, sowie einem Radikalfänger, ausgewählt aus der Gruppe bestehend aus Ascorbinsäure und/oder Alkali- und/oder Erdalkalisalze der Ascorbinsäure und/oder Ester einer oder mehrerer organischer Säuren mit Ascorbinsäure und/oder physiologisch verträgliche Derivate der Ascorbinsäure. Weiterhin betrifft die Erfindung ein Verfahren zur Herstellung solcher Zusammensetzungen sowie kosmetische oder pharmazeutische oder diätetische Formulierungen, die eine solche Zusammensetzung enthalten bzw. eine solche Zusammensetzung als Additiv verwenden.

## Beschreibung

Die Erfindung betrifft Zusammensetzungen enthaltend ein oder mehrere Phospholipid(e), einen Micellstabilisator, bestehend aus Carnitin und/oder einem physiologisch verträglichen Carnitin-Derivat und/oder Polyolen, sowie einem Radikalfänger, ausgewählt aus der Gruppe bestehend aus Ascorbinsäure und/oder Alkali- und/oder Erdalkalisalze der Ascorbinsäure und/oder Ester einer oder mehrerer organischer Säure(n) mit Ascorbinsäure und/oder physiologisch verträgliche Derivate der Ascorbinsäure. Weiterhin betrifft die Erfindung ein Verfahren zur Herstellung solcher Zusammensetzungen sowie kosmetische oder pharmazeutische oder diätetische Formulierungen, die eine solche Zusammensetzung enthalten bzw. eine solche Zusammensetzung als Additiv verwenden.

Insbesondere auf dem Gebiet der pharmazeutischen Technologie, der Diätetik als auch der kosmetischen Formulierungen besteht ein hoher Bedarf, Wirkstoffe oder Wirkstoffkombinationen sowie zersetzungsempfindliche Komponenten der jeweiligen Formulierungen langzeitstabil zu formulieren, wobei jedoch gleichzeitig insbesondere bei pharmazeutischen und diätetischen Formulierungen die physiologische Verträglichkeit der Gesamtformulierung gewährleistet werden muss. Als problematisch erweist sich vielfach die Einarbeitung geeigneter Konservierungsmittel, die insbesondere bei lipophilen Komponenten auf Grund der schwierigen Löslichkeitsverhältnisse unzureichend ist.

Stand der Technik ist, dass die Solubilisierung fettartiger Substanzen zu transparenten Systemen in den oben geschilderten Anwendungsbereichen ausschließlich mit ethoxylierten Tensiden bzw. Tensidsystemen, die einen hohen HLB-Wert besitzen und/oder Alkohol bewerkstelligt werden kann. Diese Lösungsvermittler besitzen jedoch erhebliche Nachteile. So stellen leichtflüchtige Alkohole, wie beispielsweise Ethanol, Propan-1-ol oder Isopropanol starke Zellgifte dar. Alkoxylierte Tenside sind hingegen physiologisch unverträglich und insoweit insbesondere für pharmazeutische und diätetische Formulierungen ungeeignet. Darüber hinaus müssen die alkoxylierten Tenside im Stand der Technik in einer hohen Menge eingesetzt werden, sodass die Herstellung von Konzentraten extrem erschwert ist.

DE 102 55 195 A1 beschreibt micellare wasserlösliche Konzentrate von fettartigen Substanzen mit Hilfe von Kombinationen aus Phospholipiden oder Lecithinen und hochkonzentrierten wässrigen Lösungen aus Polyolen oder Kohlenhydrate, wobei die Konzentration der Polyol- bzw. Kohlenhydratlösungen zwischen 30 und 99 Gew.-%, das Verhältnis Lecithin zu Lipid zwischen 1:1 1 und 1:10 und die Konzentration der Mischung Lecithin/Lipid in der Polyol- bzw. Kohlenhydratlösung zwischen 10 Gew.-% und 90 Gew.-% ist. Die in der DE 102 55 195 A1 offenbarten Formulierungen weisen sehr hohe Mengen an Kohlenhydraten, insbesondere Fructose auf. Dabei ergeben sich für Kohlenhydrate, insbesondere für Fructose, erhebliche physiologische Nachteile, insbesondere was den Einsatz auf dem Gebiet der pharmazeutischen Technologie und der diätetischen Formulierungen betrifft. Die beschriebenen Formulierungen bieten den zu solubilisierenden Substanzen keinen ausreichenden Schutz vor Angriffe durch Radikale als auch vor enzymatischer Zersetzung, wie sie vor allem in wässrigen Lösungen vorkommen kann. Darüber hinaus führen die in der DE 102 55 195 A1 offenbarten Formulierungen dem Körper große Mengen Zucker zu. Besonders bedenklich ist dabei der Einsatz von Fructose. Fructose wird im Körper Insulin-unabhängig aufgenommen und in den Zellen durch einmalige Phosphorylierung mittels Phosphofructokinase 10mal so schnell verstoffwechselt wie Glucose. Neuere Studien haben gezeigt, dass nach Verzehr von Fructose große Mengen Fette in der Leber gebildet und ans Blut weitergegeben werden. Die großen in der Bevölkerung nachzuweisenden Blutfettwerte sind nicht selten ursächlich mit einem hohen Verzehr an fructosehaltigen Lebensmitteln verknüpft. Hierzu zählen vor allem Kristallzucker (Saccharose). Es ist daher erforderlich, den Gehalt an Fructose in pharmazeutischen oder diätetischen Produkten so gering wie möglich zu halten. Darüber hinaus wird ein übermäßiger Verzehr von Fructose für eine Erhöhung des Cholesterinspiegels verantwortlich gemacht. Weiterhin führt eine hohe Fructosezufuhr dazu, dass die Calciumaufnahme in dem Körper gestört wird. Da Fructose im Körper sehr schnell zu Brenztraubensäuren abgebaut werden kann, ergeben sich zusätzlich indirekt Probleme bei der Calciumresorption. Der Einsatz von Produkten mit einem hohen Fructosegehalt, beispielsweise in der Diätetik oder Pharmazie, kann langfristig somit auch bezüglich des Calciumhaushaltes nachteilige Folgen für den Konsumenten haben. Die DE 102 55 195 A1 verzichtet ausdrücklich auf einen weiteren Einsatz von Konservierungsmitteln.

WO 2006/042574 A2 offenbart Solubilisatformulierungen, bestehend aus einer wässrigen Lösung von Alkali- und/oder Erdalkalisalzen der Ascorbinsäure sowie einem Emulgator mit einem HLB-Wert von 9 bis 18, insbesondere Polysorbat für Lebensmittel, Kosmetika und dergleichen.

Aufgabe der vorliegenden Erfindung ist es, langzeitstabile Zusammensetzungen bereitzustellen, die geeignet sind hydrophile als auch insbesondere lipophile Substanzen wirkungsvoll zu solubilisieren, wobei gleichzeitig eine hohe physiologische Verträglichkeit als auch Aufnahmefähigkeit der Zusammensetzung durch den Körper ermöglicht werden soll.

Darüber hinaus war es eine Aufgabe der vorliegenden Erfindung leicht handhabbare Zusammensetzungen und flüssige Formulierungen sowie ein effektives, energiesparendes und schonendes Verfahren zur Herstellung solcher Zusammensetzungen bereitzustellen.

Überraschend wurde nun gefunden, dass eine Zusammensetzung enthaltend ein oder mehrere Phospholipid(e), einen Micellstabilisator, bestehend aus Carnitin und/oder einem physiologisch verträglichen Carnitin-Derivat und/oder Polyol(en), und einen Radikalfänger, ausgewählt aus der Gruppe bestehend aus Ascorbinsäure und/oder Alkali- und/oder Erdalkalisalze der Ascorbinsäure und/oder Ester einer oder mehrerer organischer Säure(n) mit Ascorbinsäure und/oder physiologisch verträgliche Derivate der Ascorbinsäure, die vorgenannten Probleme löst.

Als wesentlichen Bestandteil enthalten die erfindungsgemäßen Zusammensetzungen ein oder mehrere Phospholipid(e). Phospholipide sind Phosphorsäuredi- oder Monoester, die wegen ihrer fettähnlichen Löslichkeitseigenschaften auf Grund der lipophilen und hydrophilen Komponenten zu den Lipiden gerechnet werden und im Organismus als Membranlipide am Aufbau von Schichtstrukturen der Membran beteiligt sind. Besonders reichlich sind Phospholipide vorhanden in der Hirnsubstanz und im Myelin. Eine besondere Gruppe der Phospholipide, die sich vom Sphingosin ableitet, sind die sogenannten Phosphosphingolipide. Die erfindungsgemäßen Zusammensetzungen enthalten jedoch bevorzugt Phospholipide, die Derivate des Glycerins darstellen und auch als Phosphoglyceride oder Phosphatide bezeichnet werden.

In der Lipid-Nomenklatur hat sich für asymmetrische Glycerin-Derivate die sogenannten stereospezifische Numerierung (Abk.: sn-, nach IUPAC/IUBMB-Regel Lip-1.13) als nützlich erwiesen, bei der die (freie oder substituierte) Hydroxy-Gruppe in Position 2 des Glycerin-Teils nach links weisend und das geminale Wasserstoff-Atom nach rechts gezeichnet und als über die Papierebene ragend gedacht werden, woraufhin das Kohlenstoff-Atom 1 des Glycerin-Teils nach oben, Kohlenstoff-Atom 3 jedoch nach unten gerichtet sind.

Formel 1:

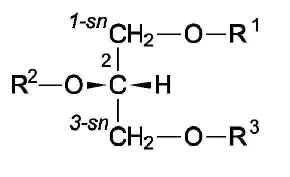

Phosphatidsäuren sind Glycerin-Derivate, die in 1-sn- und 2-Stellung mit Fettsäuren (1-sn-Position: meist gesättigt, 2-Position: meist ein- od. mehrfach ungesättigt), an Atom 3-sn dagegen mit Phosphorsäure verestert sind (Formel 1: R1, R2 = Acyl, R3 = Phosphoryl). Die Phosphatidsäuren selbst haben wahrscheinlich regulator. Funktionen für Cytoskelett, Membrantransport u. Sekretion, werden durch Phospholipase D aus Phosphatiden freigesetzt u. nur in kleinen Konz. im Gewebe gefunden; ihr Phosphat-Rest ist meist verestert mit Aminoalkoholen wie Cholin (Lecithin = 3-sn-Phosphatidylcholin) oder 2-Aminoethanol (Ethanolamin) bzw. L-Serin (Kephalin = 3-sn-Phosphatidylethanolamin bzw. -L-serin), mit myo-Inosit zu den in Geweben häufigen Phosphoinositiden [1-(3-sn-Phosphatidyl)-D-myo-inositen], mit Glycerin zu den im Fruchtwasser nachgewiesenen Phosphatidylglycerinen, die auch beim Protein-Export aus Gram-neg. Bakterien eine Rolle spielen sollen. Cardiolipine (1,3-Bisphosphatidylglycerine) sind aus Mitochondrien-Membranen des Herzmuskels isolierte Phospholipide aus zwei über Glycerin verknüpften Phosphatidsäuren. Lysophospholipide erhält man, wenn aus Phospholipiden ein Acyl-(Fettsäure-)Rest durch Phospholipase A abgespalten wird; Beispiel: Lysolecithine. Phosphatasen u. Phosphodiesterasen wie die Phospholipasen C u. D spalten Phospholipide demgegenüber an den Phosphat-Bindungen. Zu den Phospholipiden rechnet man ferner die Plasmalogene, in denen statt einer Fettsäure in 1-Stellung ein Aldehyd (in Form eines Enolethers) gebunden ist; die den Phosphatidylcholinen entsprechenden O-1-sn-Alkenyl-Verb. z. B. heißen Phosphatidalcholine. Vom Namen Plasmalogen leitet sich die Sammelbezeichnung Plasmensäuren her für Phospholipide mit einer Enolether-Gruppierung in 1-, einer (ungesättigten) Acyl-Gruppe in 2- und einer Phosphorsäure-Gruppe in 3-Stellung; die entsprechenden 1-Alkylether-Derivate heißen Plasmansäuren und desacylierte Derivate z. B. Lysoplasmenylserin. Ein weiteres Phospholipid ist der Thrombocytenaktivierende Faktor (platelet-activating factor, PAF, siehe Formel 1 mit R1 = Octadecyl, R2 = Acetyl und R3 = Phosphorylcholin). In Ciliaten-Membranen wurden Phosphonolipide entdeckt, deren Funktion möglicherweise darin besteht, ein Überleben in Anwesenheit von - selbst sezernierten - Phospholipasen zu ermöglichen.

Phosphatidyl-L-serine u. Phosphatidylinosite entstehen durch enzymatische Reaktion von L-Serin bzw. myo-Inosit mit 3-CDP-1,2-Diacyl-sn-glycerinen unter Freisetzung von Cytidin-5'-monophosphat. Phosphatidylethanolamine bilden sich bei Decarboxylierung von Phosphatidyl-L-serinen, Phosphatidylcholine wiederum durch schrittweise Methylierung der Ethanolamin-Derivate. Andererseits können freigesetztes Cholin u. Ethanolamin mit Hilfe von Cytidin-5'-triphosphat aktiviert werden zu den CDP-Derivaten und dann mit 1,2-Diacyl-sn-glycerinen unter Abspaltung von CMP zu den korrespondierenden Phosphoglyceriden reagieren.

Besonders bevorzugt enthalten die erfindungsgemäßen Zusammensetzungen Lecithin als Phospholipid. Lecithin ist die Gruppenbezeichnung für diejenigen Glycero-phospholipide, die sich aus Fettsäuren, Glycerin und Fettsäure und Cholin durch Veresterung bilden. Heute hat sich die Bezeichnung Phosphatidylcholin weitgehend durchgesetzt.

### Formel 2:

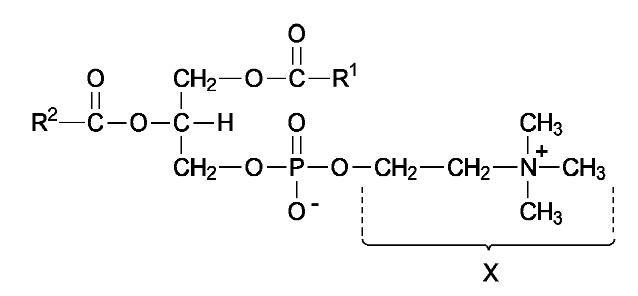

Allg. Struktur der Lecithine; R1, R2: typischerweise unverzweigte aliphatische Reste mit 15 oder 17 Kohlenstoff-Atomen und bis zu 4 cis-Doppelbindungen.

Die in der Natur vorkommenden Lecithine sind, wie die eng verwandten Kephaline, Derivate der 1,2-Diacyl-sn-glycerin-3-phosphorsäuren (Phosphatidsäuren; X = H; R2-CO-O- in Fischer-Projektion nach links weisend). Beim b-Lecithin ist der mit Cholin veresterte Phosphorsäure-Rest an die mittelständige Hydroxy-Gruppe eines Glycerids gebunden. Aus der Verschiedenheit der Fettsäure-Reste R1 u. R2 ergibt sich eine große Zahl verschiedener Lecithine. Bei Extraktionen aus biologischen Material erhält man immer Gemische. So enthält eine Lecithin-Fraktion aus Sojabohnen z. B. Palmitinsäure, Stearinsäure, Palmitoleinsäure, Oleinsäure, Linolsäure und Linolensäure. Normalerweise ist die gesättigte Fettsäure mit der primären, die ungesättigte mit der sekundären Hydroxy-Gruppe des Glycerins verestert.

In einer bevorzugten Ausführungsform der vorliegenden Erfindung enthalten die erfindungsgemäßen Zusammensetzungen Phospholipid, das aus Soda und/oder Raps und/oder Ei gewonnen wurde.

Weiter bevorzugt enthalten die erfindungsgemäßen Zusammensetzungen hydrierte und/oder hydrolysierte und/oder hydroxylierte Phospholipide.

Das/Die Phospholipid(e) liegen in den erfindungsgemäßen Zusammensetzungen vorzugsweise in einer Menge von 0,4 bis 50 Gew.-%, weiter bevorzugt von 0,7 bis 40 Gew.-%, besonders bevorzugt von 2 bis 20 Gew.-% und insbesondere von 2,5 bis 15 Gew.-%, jeweils bezogen auf die Gesamtzusammensetzung, vor.

Ein weiterer wesentlicher Bestandteil der erfindungsgemäßen Zusammensetzung ist ein Radikalfänger, ausgewählt aus der Gruppe bestehend aus Ascorbinsäure und/oder Alkali- und/oder Erdalkali-Salze der Ascorbinsäure und/oder Ester einer oder mehrerer organischer Säure(n) mit Ascorbinsäure und/oder physiologisch verträgliche Derivate der Ascorbinsäure. Vorzugsweise enthalten die erfindungsgemäßen Zusammensetzungen Alkali- und/oder Erdalkalisalze der Ascorbinsäure. Hierbei sind Radikalfänger, ausgewählt aus der Gruppe bestehend aus Calciumascorbat, Natriumascorbat, Kaliumascorbat oder Magnesiumascorbat besonders bevorzugt. In einer weiteren bevorzugten Ausführungsform enthalten die erfindungsgemäßen Zusammensetzungen Ester, vorzugsweise Monoester, organischer Säuren mit Ascorbinsäure. In einer bevorzugten Ausführungsform ist der Radikalfänger ein C₈-C₃₀-Fettsäureester, vorzugsweise C₈-C₃₀-Fettsäuremonoester, weiter bevorzugt C₁₂-C₂₂-Fettsäuremonoester, der Ascorbinsäure, besonders bevorzugt Ascorbylpalmitat oder Ascorbylstearat oder beliebige Mischungen hiervon.

Die/Der Radikalfänger liegt in einer besonderen Ausführungsform in einer Menge von 1 - 95 Gew.-%, vorzugsweise von 4 - 50 Gew.-%, besonders bevorzugt von 5 - 45 Gew.-% und insbesondere von 7 - 25 Gew.-%, jeweils bezogen auf die Gesamtzusammensetzung, vor.

Als weiteren wesentlichen Bestandteil enthält die erfindungsgemäße Zusammensetzung einen Micellstabilisator, bestehend aus Carnitin und/oder einem physiologisch verträglichen Carnitin-Derivat und/oder Polyol(en).

Carnitin (3-Hydroxy-4-(trimethylammonio)-buttersäurebetain) bildet farblose hygroskopische Kristalle und ist in Wasser leicht löslich. Die L-Form des Carnitins ist in tierischen Geweben weit verbreitet und ein charakteristischer Bestandteil der gestreiften Muskulatur. In einer bevorzugten Ausführungsform enthalten die erfindungsgemäßen Zusammensetzungen L-Carnitin und/oder Carnitin-Derivate, ausgewählt aus L-Carnitintartrat oder Acetyl-L-Carnitin.

Polyole im Rahmen der vorliegenden Erfindung umfassen alle organischen Moleküle, die mindestens 2, vorzugsweise 3 Hydroxygruppen aufweisen. Weiterhin ist im Rahmen der vorliegenden Erfindung der erfindungsgemäß einzusetzenden Radikalfänger, ausgewählt aus der Gruppe bestehend aus Ascorbinsäure und/oder Alkali- und/oder Erdalkalisalze der Ascorbinsäure und/oder Ester einer oder mehrerer organischen Säure(n) mit Ascorbinsäure und/oder physiologisch verträgliche Derivate der Ascorbinsäure, kein Polyol. Bevorzugt enthalten die erfindungsgemäßen Zusammensetzungen aliphatische Polyole mit Alkylkettenlängen, die 3 bis 10, besonders bevorzugt 3 bis 6 Kohlenstoffatome umfassen. In einer bevorzugten Ausführungsform ist das Polyol ausgewählt aus der Gruppe der C₃-C₆-Di-, -Tri-, -Tetra-, -Penta- oder - Hexaole, vorzugsweise Glycerin, Pentite, weiter bevorzugt Xylit oder Hexit, insbesondere Sorbit, Mannit, Gulit oder Inositol.

In einer weiteren bevorzugten Ausführungsform ist das Polyol ausgewählt aus der Gruppe der Kohlenhydrate, vorzugsweise der Monosaccharide, Disaccharide und/oder Oligosaccharide, insbesondere Malitol, Maltodextrin, Traubenzucker, Glucose und/oder Pektin und/oder dessen physiologisch akzeptable Derivate.

Bevorzugte erfindungsgemäße Zusammensetzungen sind im Wesentlichen frei von Fructose, bevorzugt liegt der Fructose-Gehalt unterhalb von 5 Gew.-%, weiter bevorzugt unterhalb von 2 Gew.-%, insbesondere unterhalb von 1 Gew.-%, weiter bevorzugt unterhalb von 0,05 Gew.-%, jeweils bezogen auf die gesamte Zusammensetzung. Spezielle Zusammensetzungen gemäß der vorliegenden Erfindung sind frei von Fructose.

Das Carnitin und/oder ein physiologisch verträgliches Carnitin-Derivat liegt in einer bevorzugten Ausführungsform in einer Menge von 1 bis 96,8 Gew.-%, vorzugsweise von 30 bis 60 Gew.-%, besonders bevorzugt von 5 bis 55 Gew.-% und insbesondere von 9 bis 25 Gew.-%, jeweils bezogen auf die Gesamtzusammensetzung in den erfindungsgemäßen Zusammensetzungen vor.

Das/Die Polyol(e) liegen vorzugsweise in einer Menge von 1,8 bis 95 Gew.-%, weiter bevorzugt von 3,5 bis 60 Gew.-%, besonders bevorzugt von 5 bis 40 Gew.-% und insbesondere von 9 bis 25 Gew.-%, jeweils bezogen auf die gesamte Zusammensetzung vor.

Die erfindungsgemäßen Zusammensetzungen zeichnen sich durch eine besonders gute physiologische Verträglichkeit aus. In einer bevorzugten Ausführungsform weisen die erfindungsgemäßen Zusammensetzungen unterhalb von 5 Gew.-%, vorzugsweise unterhalb von 2,5 Gew.-%, weiter bevorzugt unterhalb von 1 Gew.-%, insbesondere sind sie jedoch im Wesentlichen frei von C₁-C₄-Monoalkoholen, insbesondere im Wesentlichen frei von Ethanol oder C₃-Monool und/oder im Wesentlichen frei von künstlichen Tensiden oder Emulgatoren, insbesondere im Wesentlichen frei von alkoxylierten Alkoholen, Säuren oder Aminen. In einer speziellen Ausführungsform sind die Zusammensetzungen frei von C₁-C₄-Monoalkoholen und/oder künstlichen Tensiden oder Emulgatoren oder alkoxylierten Alkoholen, Aminen oder Säuren.

Die erfindungsgemäßen Zusammensetzungen zeichnen sich dadurch aus, dass sie in der Lage sind, insbesondere lipophile Stoffe zu solubilisieren. Durch die Einarbeitung lipophiler Stoffe können diese nicht nur in einem wässrigen System solubilisiert werden, sondern zusätzlich hervorragend stabilisiert und vor enzymatischen und oxidativen Angriffen geschützt werden. Prinzipiell können in die erfindungsgemäßen Zusammensetzungen lipophile Stoffe jeglicher Art, insbesondere Stoffe, die in der pharmazeutischen Technologie, Pharmazie, Pharmakologie, Diätetik und Kosmetik Verwendung finden, eingearbeitet werden. In einer bevorzugten Ausführungsform enthalten die erfindungsgemäßen Zusammensetzungen daher zusätzlich einen oder mehrere lipophile(n) solubilisierbare(n) Stoff(e) ausgewählt aus der Gruppe der C_{1O}-C₃₀-Fettsäuren, Wachse oder deren Ester, Paraffine, C₁₀-C₃₀-Fettalkohole, C_{1O}-C₃₀-Fettaldehyde, Triglyceride, Öle, Isoprenoide, Terpene, Citral, Menthol Campher, Bisabolol, Cholesterin oder deren Derivate, Vitamin D, Testosteron, Östrogen, Progesteron, Cortisol, Aldosteron und jeweils deren physiologisch verträglichen/akzeptablen Derivate, Ethinylestradiol, Sitosterole, Vitamin A, Vitamin K, Vitamin E, Tocopherole, Tocotrienole, Carotinoide, Beta-Carotin, Lutein, Zeaxanthin oder deren physiologisch verträglichen Derivate, Coenzym Q10 und polare Lipide, Ceramide, Sphingilipide, Glykolipide, Amine, Aminosäuren, Peptide oder deren Derivate, Siliconöle sowie lipophile UV-A und UV-B Filter.

Als besonders geeignet haben sich die erfindungsgemäßen Zusammensetzungen erwiesen für die Solubilisierung und Stabilisierung pharmazeutischer Wirkstoffe, die sowohl in flüssiger Form als auch in fester Form verabreicht werden können. Die erfindungsgemäße Zusammensetzung hat hierbei zusätzlich den Vorteil, dass die pharmazeutischen Wirkstoffe sehr gut aufgenommen werden können. Die einen pharmazeutischen Wirkstoff enthaltenden Zusammensetzungen können als solches bereits die galenische Formulierung darstellen oder in fester oder flüssiger Form einer weiteren galenischen Formulierung zugefügt werden.

Bevorzugt enthalten die Zusammensetzungen daher einen pharmazeutischen Aktivstoff.

In einer bevorzugten Ausführungsform enthalten die erfindungsgemäßen Zusammensetzungen zusätzlich mindestens ein Mitglied, ausgewählt aus der Gruppe enthaltend Melatonin, 5-Hydroxytryptophan, Tryptophan, Serotonin, Dopamin oder L-Dopa.

In einer weiteren Ausführungsform enthält die erfindungsgemäße Zusammensetzung zusätzlich Vitamine, ausgewählt aus der B-Reihe.

Bevorzugt sind Vitamin B1, Riboflavin, Pyridoxin, Pyridoxamin oder Pyridoxal enthalten.

Die zusätzlichen solubilisierbaren Stoffe sind in den erfindungsgemäßen Zusammensetzungen in einer Menge von 0 bis 50 Gew.-%, vorzugsweise von 1 bis 35 Gew.-%, besonders bevorzugt von 3 bis 30 Gew.-% und insbesondere von 5 bis 25 Gew.-%, jeweils bezogen auf die gesamte Zusammensetzung, enthalten.

Insbesondere für die Verarbeitung der erfindungsgemäßen Zusammensetzung zu getrockneten Pulvern hat sich der Zusatz anorganischer oder organischer Säuren und/oder deren Salze oder auch der Zusatz von Stärke als besonders geeignet herausgestellt. In einer bevorzugten Ausführungsform enthalten die erfindungsgemäßen Zusammensetzungen daher zusätzlich eine anorganische oder organische Säure und/oder deren Salze, bevorzugt ausgewählt aus der Gruppe enthaltend Citronensäure, Gluconsäure, Calcium- und/oder Magnesiumcitrat, Calcium-, Magnesium- oder Natriumgluconat, Natrium- oder Calciumhydrogencarbonat oder -hydrogenphosphat.

Als Stärke können die erfindungsgemäßen Zusammensetzungen vorzugsweise Mais-, Reis- oder Kartoffelstärke enthalten.

Ein erheblicher Vorteil der erfindungsgemäßen Zusammensetzungen liegt darin, dass sie sich einfach als Feststoffe verarbeiten lassen. Im Gegensatz zu herkömmlichen Systemen lassen sich mit den erfindungsgemäßen Zusammensetzungen feinteilige Pulver herstellen, so dass es nicht nur möglich ist, solubilisierbare Stoffe, insbesondere lipophile Stoffe in hoher Konzentration stabil und oxidationsgeschützt bereitzustellen, sondern gleichzeitig eine geeignete und gute Handhabbarkeit zur Verfügung gestellt ist. Vorzugsweise liegen die erfindungsgemäßen Zusammensetzungen als Feststoff, insbesondere als Pulver oder Granulat oder als gepresster Formkörper vor. Die Pulver weisen dabei eine mittlere Teilchengröße von 0,1 bis 5000 µm, vorzugsweise von 100 bis 800 µm auf. Gemessen wurden die mittlere Teilchengröße der Pulver mit einem Coulter counter.

Gepresste Formkörper, wie beispielsweise Tabletten, können die dem Fachmann geläufigen zusätzlichen Komponenten, wie etwa Sprengmittel aufweisen.

Insbesondere dann, wenn die erfindungsgemäßen Zusammensetzungen zusätzlich temperaturempfindliche Aktivstoffe enthalten, hat sich das Sprühtrocknungsverfahren zur Gewinnung des Feststoffs als besonders geeignet für die erfindungsgemäßen Zusammensetzungen erwiesen. Vorzugsweise liegen die erfindungsgemäßen Zusammensetzungen daher als sprühgetrockneter Feststoff, vorzugsweise als sprühgetrocknetes Pulver vor.

Ein weiterer Gegenstand der vorliegenden Erfindung sind flüssige wässrige Formulierungen, die die erfindungsgemäße Zusammensetzung enthalten. Vorzugsweise liegen diese flüssigen Formulierungen als Emulsion oder Dispersion vor. Der Wassergehalt der flüssigen wässrigen Formulierung liegt in einer bevorzugten Ausführungsform von 0,1 bis 81 Gew.-%, vorzugsweise von 3 bis 75 Gew.-% und insbesondere von 8 bis 30 Gew.-%, jeweils bezogen auf die gesamte wässrige Formulierung, vor.

Die erfindungsgemäßen flüssigen wässrigen Formulierungen bilden regelmäßig Micellen aus, die insbesondere geeignet sind liphophile Stoffe zu solubilisieren.

Die Ausbildung von Micellen macht es auch möglich, konzentrierte flüssige Formulierungen herzustellen, die beispielsweise einen Wassergehalt unterhalb von 40 Gew.-% aufweisen. In einer bevorzugten Ausführungsform liegen die flüssigen wässrigen Formulierungen als micellares Konzentrat, vorzugsweise als micellare Emulsion vor. Die Micellen weisen dabei bevorzugt eine mittlere Größe von 3 bis 250 nm, vorzugsweise von 10 bis 120 nm auf. Die flüssigen wässrigen Formulierungen gemäß der vorliegenden Erfindung sind bevorzugt transparent und liegen insbesondere als transparente Emulsion vor.

Die Herstellung der flüssigen wässrigen Formulierung erfolgt in einem sogenannten Eintopf-Verfahren, bei dem die Komponenten der erfindungsgemäßen Zusammensetzung in Wasser eingerührt und vermischt werden. Die Vermischung kann je nach Zusammensetzung mit dem Fachmann geläufigen Rührwerkzeugen oder mit hochscherenden Vorrichtungen, die dem Fachmann geläufig sind, behandelt werden. Bevorzugt ist der Einsatz von Ultraturax-Geräten oder von Hochdruck-Homogenisatoren. Für die Herstellung transparenter flüssiger Formulierungen hat sich die Homogenisierung mittels eines Hochdruck-Homogenisators als besonders geeignet herausgestellt.

Ein weiterer Gegenstand der vorliegenden Erfindung ist ein Verfahren zur Herstellung der erfindungsgemäßen Zusammensetzung, wobei eine erfindungsgemäße flüssige wässrige Formulierung einer Sprühtrocknung zugeführt wird.

Für die Sprühtrocknung können handelsübliche Sprühtrocknungsvorrichtungen verwendet werden. Dazu werden die flüssigen wässrigen Formulierungen gemäß der vorliegenden Erfindung der Sprühtrockenvorrichtung zugeführt und in der Vorrichtung versprüht. Es hat sich dabei als vorteilhaft herausgestellt, dass die flüssige wässrige Formulierung zunächst mit einem HochdruckHomogenisator homogenisiert und anschließend der Sprühtrocknung zugeführt wird. Die Sprühtrocknung ist ein kontinuierlich durchführbares Verfahren zur Trocknung von Lösungen, Suspensionen oder pastösen Massen. Mittels einer Düse (durch Flüssigkeitsdruck oder Pressluft bzw. Inertgas betrieben) oder rotierenden Zerstäuberscheiben (400-50000 U/min) wird das zu trocknende Gut in einen Trockengasstrom eingebracht, der es in Bruchteilen einer Sekunde zu einem feinen Pulver trocknet. Das Trocknen kann in Richtung mit dem Sprühstrahl oder gegen den Sprühstrahl strömen (Gleichstrom-, Gegenstromverfahren), je nach Bauart oder Verwendungszweck. Die Sprüheinrichtung befindet sich bevorzugt am oberen Teil eines Sprühturms, das anfallende Trockengut wird meist durch einen Zyklonabscheider vom Luftstrom getrennt und kann dort entnommen werden. Die erfindungsgemäße wässrige Formulierung wird vorzugsweise am oberen Ende eines Sprühtrockenturms über Sprühdüsen in die Sprühtrocknungsvorrichtung eingebracht. Die Sprühtrocknung erfolgt bevorzugt, indem aus einem unteren Bereich des Sprühtrockenturms Gas durch den Trockenturm geführt wird. Die Sprühtrocknung erfolgt dabei vorzugsweise bei einer Trockengas-Temperatur von 5 bis 200 °C, vorzugsweise bei 30 bis 120 °C. Das Sprühtrockenverfahren ist ein besonders schonendes Verfahren, mit dem die erfindungsgemäßen Zusammensetzungen ökonomisch aber auch stabil und unbeschadet erhalten werden können. Es hat sich gezeigt, dass sich pulverförmige oder granulatförmige Produkte der erfindungsgemäßen Zusammensetzung, insbesondere sprühgetrocknete Feststoffe der erfindungsgemäßen Zusammensetzung, durch ein Sprühtrockenverfahren hergestellt werden können, das mittels der Strahlschicht-Technologie betrieben wird. Grundlage der Strahlschicht-Technologie ist die Fluidisierung von Partikeln durch nach oben strömendes Trockengas (Prozessgas). Anders als bei Wirbelschichtprozessen tritt die Luft nicht von unten durch einen Siebboden in die Prozesskammer, sondern durch im unteren Bereich liegende Längsspalte. In einer vorteilhaften Ausgestaltung des erfindungsgemäßen Verfahrens ist der Querschnitt der Prozesskammer der Sprühtrockenvorrichtung nach oben deutlich aufgeweitet. Dadurch sinkt die Anströmgeschwindigkeit in der Prozesskammer stark ab. Bevorzugt wird das Trockengas seitlich durch Längsspalte in die Prozesskammer eingeführt. Die Spaltbreite kann dabei beispielsweise durch walzenförmige Anordnungen eingestellt werden. Je nach Größe der Sprühtrockenvorrichtung und dem Trockengasdruck kann die Spaltbreite über einem großen Bereich variieren. Bevorzugt beträgt die Spaltbreite 0,2 bis 200 mm, besonders bevorzugt 0,3 bis 100 mm, insbesondere 0,4 bis 60 mm, beispielsweise 0,4 bis 55 mm. In einer weiteren bevorzugten Ausführungsform des erfindungsgemäßen Verfahrens befinden sich oberhalb des Trockengaseinströmspalts Platten, die zu beiden Seiten des Spalts, vorzugsweise regelmäßig angeordnet sind, so dass die Breite des Trockengaseinströmspaltes durch die Platten weiter verengt wird. Das Trockengas wird dabei nicht ausschließlich durch den verbleibenden Spalt geführt, sondern kann gemäß einer bevorzugten Ausgestaltung durch in die Platten eingelassene Kanäle strömen. Die Kanäle sind dabei vorteilhafterweise Bohrungen in den Platten, die bevorzugt mit einem abweichenden Winkel bezüglich der Hauptstromrichtung durch den Spalt in die Platten eingelassen sind. Vorzugsweise weisen die linearen Kanäle in den Platten einen Winkel von 0 bis 75°, vorzugsweise 10 bis 65°, weiter bevorzugt 20 bis 55° und insbesondere 30 bis 50°, beispielsweise 45° angeordnet, wobei 0° parallel zur Hauptstromrichtung durch den Spalt ist und 90° parallel zur Längsausrichtung der Platte ist. Die Trockengaskanäle in den Platten können dabei regelmäßig oder vorteilhafterweise unregelmäßig angeordnet werden. Die schräg eingelassenen Trockengaskanäle in den Platten haben den Vorteil, dass das gegen die Platten strömende Trockengas über die Kanäle in die Prozesskammer des Sprühtrockners einströmen kann. Es hat sich überraschend gezeigt, dass diese Anordnung des Sprühtrockners eine sehr schonende Trocknung für die teilweise sehr temperaturempfindlichen Produkte darstellt. Es wurde beobachtet, dass das Trockengut langsamer abkühlt und die erfindungsgemäß hergestellten sprühgetrockneten Zusammensetzungen eine höhere Kristallinität und bessere Rieselfähigkeit und damit eine deutlich verbesserte Verabreitbarkeit der sprühgetrockneten erfindungsgemäßen Zusammensetzungen zeigt. In einer weiter bevorzugten Ausgestaltung sind die vorgenannten Platten an ihren Enden zum Trockengaseinströmspalt hin abgeschrägt, so dass der Querschnitt in Richtung Prozesskammer aufgeweitet ist. Dies führt zu einem zusätzlichen Absinken der Anströmgeschwindigkeit in der Prozesskammer und beschleunigt damit die vorteilhaften Eigenschaften des erfindungsgemäßen Verfahrens. Die Platten bestehen vorzugsweise aus Metallen oder thermisch-belastbaren Kunststoffen. Bevorzugt bestehen die Platten aus V4A-Stahl. Die Materialstärke und die Anzahl der Kanäle in den Platten ist abhängig von den Auslegungen des Sprühtrockners und der Prozesskammer. Der durch die erfindungsgemäß einzusetzenden Platten verbleibende Trockengaseinströmspalt weist vorzugsweise eine Spaltbreite von 4 bis 55 mm, besonders bevorzugt von 6 bis 40 mm, insbesondere von 10 bis 35 mm auf.

In einer bevorzugten Ausführungsform des erfindungsgemäßen Verfahrens enthält die flüssig-wässrige Formulierung ein oder mehreren Phospholipid(e), ein oder mehreren Polyol(e) und/oder Carnitin oder ein Carnitin-Derivat, einen oder mehrere solubilisierbare(n) Stoff(e) und einen oder mehrere Radikalfänger, ausgewählt aus der Gruppe bestehend aus Ascorbinsäure und/oder Alkali und/oder Erdalkali-Salze der Ascorbinsäure und/oder Ester einer oder mehrerer organischer Säure(n) mit Ascorbinsäure und/oder physiologisch verträgliche Derivate der Ascorbinsäure. Insbesondere für das erfindungsgemäße Verfahren zur Herstellung der erfindungsgemäßen Zusammensetzungen hat sich als vorteilhaft herausgestellt, dass zunächst die erfindungsgemäße wässrige flüssige Formulierung zusammen mit einer weiteren wässrigen Formulierung A, enthaltend eine organische oder anorganische Säure oder deren Salze, bevorzugt ein Polycarbonsäure-Salz, vorzugsweise Citronensäuresalz, insbesondere Calcium und/oder Magnesiumcitrat oder ein Gluconsäuresalz, insbesondere Natrium-, Magnesium- oder Calciumgluconat der Sprühtrocknung zugeführt wird. Weiterhin kann bevorzugt die wässrige flüssige Formulierung zusammen mit einer weiteren wässrigen Formulierung A, enthaltend Stärke, insbesondere Reis-, Mais- und/oder Kartoffelstärke der Sprühtrocknung zugeführt werden.

In einer bevorzugten Ausgestaltung des erfindungsgemäßen Verfahrens erfolgt die Zuführung der wässrigen Formulierung A separat von den zu trocknenden erfindungsgemäßen flüssigen Formulierungen. Die separate Zuführung der Formulierung A kann hierzu über eine zweite Düse, die getrennt von der Düse angeordnet ist, die die erfindungsgemäße flüssige Formulierung eindüst, dem Sprühtrockner zugeführt werden. Die Düsen sind dabei vorteilhafterweise so angeordnet, dass in dem Sprühtrockner eine hinreichende Vermischung der erfindungsgemäßen flüssigen Formulierung mit der wässrigen Formulierung A erfolgt. In einer weiteren Ausgestaltung kann das Eindüsen der wässrigen Formulierung A und der erfindungsgemäßen flüssigen Formulierung auch über eine einzelne Düse erfolgen, wobei es sich als vorteilhaft herausgestellt hat, wenn die wässrige Formulierung A und die erfindungsgemäße flüssige Formulierung über zwei getrennte Zuläufe zunächst zusammengeführt und anschließend sofort in den Sprühtrockner eingedüst wird.

Die Düsen im Prozessraum des Sprühtrockners können von oben nach unten oder von unten nach oben sprühen. Bevorzugt wird durch eine zentrale Anordnung der Düsen an der Stelle des höchsten Energieeintrags gesprüht.

Die Hinzufügung der separaten Formulierung A zu den erfindungsgemäßen wässrigen flüssigen Formulierungen während des Sprühtrocknungsverfahrens hat gezeigt, dass besonders rieselfähig, leicht verarbeitbare Pulver erhalten werden können.

Die erfindungsgemäßen Zusammensetzungen oder auch die erfindungsgemäßen flüssigen Formulierungen eignen sich insbesondere für kosmetische oder pharmazeutische oder diätetische Zusammensetzungen. Sie können dort als Additiv in der Gesamtformulierung verwendet werden oder bereits die gesamte kosmetische oder pharmazeutische oder diätetische Zusammensetzung darstellen. Ein weiterer Gegenstand der vorliegenden Erfindung ist daher eine kosmetische oder pharmazeutische oder diätetische Zusammensetzung enthaltend eine erfindungsgemäße Zusammensetzung oder eine erfindungsgemäße flüssige Formulierung. Darüber hinaus ist ein weiterer Gegenstand der vorliegenden Erfindung die Verwendung der erfindungsgemäßen Zusammensetzung oder der erfindungsgemäßen flüssigen Formulierung als Additiv in kosmetischen, pharmazeutischen oder diätetischen Formulierungen.

Die erfindungsgemäßen Zusammensetzungen oder erfindungsgemäßen flüssigen Formulierungen werden als Einzelportionen üblicherweise vertrieben. Ein weiterer Gegenstand der vorliegenden Erfindung ist daher eine Einzelportion enthaltend eine erfindungsgemäße Zusammensetzung oder eine erfindungsgemäße flüssige Formulierung. Vorzugsweise liegt die Einzelportion in Form einer Kapsel oder einer Ampulle vor. Die erfindungsgemäßen Einzelportionen enthalten die erfindungsgemäße Zusammensetzung vorzugsweise in einer Menge von 0,1 bis 100 g. Werden pulverförmige erfindungsgemäße Zusammensetzungen in Kapseln portioniert, so beträgt die Zusammensetzungsmenge bevorzugt 0,1 bis 0,75 g. Wird die erfindungsgemäße wässrige flüssige Formulierung in Ampullen portioniert, so liegt diese vorzugsweise in einer Menge von 5 bis 100 g vor.

### Ausführungsbeispiele:

### Beispiel 1:

Es wurde die folgende erfindungsgemäße Zusammensetzung hergestellt:

**Tabelle 1:**

| Rohstoff | Menge in Gewichtsprozent |
|---|---|
| Sojaphospholipid | 4,3 |
| Glucose | 38,0 |
| Ascorbinsäure | 15,2 |
| mittelkettige Triglyceride* | 19,7 |
| Wasser | ad 100 |

| | |
|---|---|
| * Veresterungsprodukte aus Glycerin mit einer Mischung aus Caprylsäure und Caprinsäure | |

Die in der Tabelle 1 aufgeführte Zusammensetzung wird mittels Hochdruckhomogenisator homogenisiert. Es entsteht ein transparentes Micellenkonzentrat.

### Beispiel 2:

50 g des erhaltenen micellaren Konzentrates werden mit 100 ml Wasser verdünnt. Diese Lösung wird anschließend mit einer 15 %igen wässrigen Lösung aus Calcium- und Magnesiumcitrat im Verhältnis 2:1 im Sprühtrockner verarbeitet. Man erhält ein farbloses, wasserlösliches Pulver.

### Beispiel 3:

50 g des unter Beispiel 1 aufgeführten erhaltenen micellaren Konzentrates werden mit 100 ml Wasser verdünnt. Diese Lösung wird mit einem 20% Stärke-Konzentrat bestehend aus Maisstärke im Sprühtrockner verarbeitet. Man erhält ein farbloses wasserunlösliches Pulver.

### Beispiel 4:

Erfindungsgemäßes Micellenkonzentrat:

**Tabelle 2:**

| Menge in Gewichtsprozent | Rohstoff |
|---|---|
| 5 Gew.-% | Calciumascorbat |
| 5 Gew.-% | Carnitin |
| 40 Gew.-% | Glucose |
| 5 Gew.-% | Sojaphospholipid |
| 20 Gew.-% | Vitamin E |
| ad 100 | Wasser |

### Beispiel 5:

Erfindungsgemäßes Micellenkonzentrat:

**Tabelle 3:**

| Menge in Gewichtsprozent | Rohstoff |
|---|---|
| 5 Gew.-% | Calciumascorbat |
| 5 Gew.-% | Carnitin |
| 40 Gew.-% | Glucose |
| 5 Gew.-% | Sojaphospholipid |
| 20 Gew.-% | Coenzym Q10 |
| ad 100 | Wasser |

### Beispiel 6:

Erfindungsgemäßes Micellenkonzentrat:

**Tabelle 4:**

| Menge in Gewichtsprozent | Rohstoff |
|---|---|
| 5 Gew.-% | Calciumascorbat |
| 5 Gew.-% | Carnitin |
| 40 Gew.-% | Glucose |
| 5 Gew.-% | Sojaphospholipid |
| 20 Gew.-% | Testosteron |
| ad 100 | Wasser |

Die in den Beispielen 3 bis 6 aufgeführten Micellenkonzentrate werden entsprechend Beispiel 3 einem Sprühtrockenverfahren, das auf Basis der Strahlschicht-Technologie arbeitet, zugeführt. Es konnten rieselfähige, leicht verarbeitbare Pulver erhalten werden.

## Patentansprüche

1. Zusammensetzung enthaltend
ein oder mehrere Phospholipid(e),
einen Micellstabilisator, bestehend aus Carnitin und/oder einem physiologisch verträglichen Carnitin-Derivat und/oder Polyol(en), und
einen Radikalfänger, ausgewählt aus der Gruppe bestehend aus Ascorbinsäure und/oder Alkali- und/oder Erdalkalisalze der Ascorbinsäure und/oder Ester einer oder mehrerer organischer Säure(n) mit Ascorbinsäure und/oder physiologisch verträgliche Derivate der Ascorbinsäure.

2. Zusammensetzung gemäß Anspruch 1, **dadurch gekennzeichnet, dass** sie im Wesentlichen frei ist von Fructose.

3. Zusammensetzung gemäß einem der Ansprüche 1 oder 2, **dadurch gekennzeichnet, dass** sie zusätzlich einen oder mehrere lipophile solubilisierbare(n) Stoff(e) ausgewählt aus der Gruppe der C₁₀-C₃₀-Fettsäuren, Wachse oder deren Ester, Paraffine, C₁₀-C₃₀-Fettalkohole, C₁₀-C₃₀-Fettaldehyde, Triglyceride, Öle, Isoprenoide, Terpene, Citral, Menthol Campher, Bisabolol, Cholesterin oder deren Derivate, Vitamin D, Testosteron, Östrogen, Progesteron, Cortisol, Aldosteron und jeweils deren physiologisch verträglichen/akzeptablen Derivate, Ethinylestradiol, Sitosterole, Vitamin A, Vitamin K, Vitamin E, Tocopherole, Tocotrienole, Carotinoide, Beta-Carotin, Lutein, Zeaxanthin oder deren physiologisch verträglichen Derivate, Coenzym Q10 und polare Lipide, Ceramide, Sphingilipide, Glykolipide, Amine, Aminosäuren, Peptide oder deren Derivate, Siliconöle sowie lipophile UV-A und UV-B Filter, enthält.

4. Zusammensetzung gemäß einem der Ansprüche 1 bis 3, **dadurch gekennzeichnet, dass** zusätzlich mindestens ein Mitglied ausgewählt aus der Gruppe enthaltend Melatonin, 5-Hydroxytroptophan, Tryptophan, Seratonin, Dopamin oder L-Dopa enthalten ist.

5. Zusammensetzung gemäß einem der Ansprüche 1 bis 4, **dadurch gekennzeichnet, dass** ein Vitamin ausgewählt aus der B-Reihe zusäztlich enthalten ist.

6. Zusammensetzung gemäß einem der Ansprüche 1 bis 5, **dadurch gekennzeichnet, dass** sie zusätzlich eine anorganische oder organische Säure und/oder deren Salze enthält, bevorzugt ausgewählt aus der Gruppe enthaltend Citronensäure, Gluconsäure, Calcium und/oder Magnesiumcitrat, Calcium-, Magnesium- oder Natriumgluconat, Natrium- oder Calciumhydrogencarbonat oder -hydrogenphosphat.

7. Zusammensetzung gemäß einem der Ansprüche 1 bis 6, **dadurch gekennzeichnet, dass** sie zusätzlich Stärke, vorzugsweise Mais-, Reis- oder Kartoffelstärke enthält.

8. Zusammensetzung gemäß einem der Ansprüche 1 bis 7, **dadurch gekennzeichnet, dass** sie als Feststoff, vorzugsweise als Pulver oder Granulat oder als gepresster Formkörper vorliegt.

9. Flüssige wässerige Formulierung enthaltend eine Zusammensetzung gemäß einem der Ansprüche 1 bis 8.

10. Verfahren zur Herstellung einer Zusammensetzung gemäß einem der Ansprüche 1 bis 8, **dadurch gekennzeichnet, dass** eine flüssige wässrige Formulierung gemäß Anspruch 9 einer Sprühtrocknung zugeführt wird.

11. Verfahren nach Anspruch 10, **dadurch gekennzeichnet, dass** die wässrige flüssige Formulierung zusammen mit einer weiteren wässrigen Formulierung A, enthaltend ein Polycarbonsäuresalz, vorzugsweise Citronensäuresalz, insbesondere Calcium- und/oder Magnesiumcitrat oder Gluconsäuresalz, insbesonder Na-, Mg- oder Ca-Gluconate, der Sprühtrocknung zugeführt wird.

12. Verfahren nach einem der Ansprüche 10 oder 11, **dadurch gekennzeichnet, dass** die wässrige flüssige Formulierung zusammen mit einer weiteren wässrigen Formulierung A, enthaltend Stärke, insbesondere Reis-, Mais- und/oder Kartoffelstärke, der Sprühtrocknung zugeführt wird.

13. Kosmetische oder pharmazeutische oder diätetische Zusammensetzung enthaltend eine Zusammensetzung gemäß einem der Ansprüche 1 bis 8 oder eine flüssige Formulierung gemäß Anspruch 9.

14. Verwendung der Zusammensetzung gemäß einem der Ansprüche 1 bis 8 oder eine flüssige Formulierung gemäß Anspruch 9 als Additiv in kosmetischen, pharmazeutischen oder diätetischen Formulierungen.
